(19) Europäisches Patentamt
European Patent Office
Office européen des brevets

(11) Veröffentlichungsnummer : **0 347 692 B1**

# (12) EUROPÄISCHE PATENTSCHRIFT

(45) Veröffentlichungstag der Patentschrift : **26.08.92 Patentblatt 92/35**

(51) Int. Cl.[5] : **C07C 47/55,** C07C 45/63

(21) Anmeldenummer : **89110560.3**

(22) Anmeldetag : **10.06.89**

## (54) Verfahren zur Herstellung von fluorsubstituierten Benzaldehyden.

(30) Priorität : **22.06.88 DE 3820979**

(43) Veröffentlichungstag der Anmeldung : **27.12.89 Patentblatt 89/52**

(45) Bekanntmachung des Hinweises auf die Patenterteilung : **26.08.92 Patentblatt 92/35**

(84) Benannte Vertragsstaaten : **BE CH DE FR GB IT LI NL**

(56) Entgegenhaltungen :
**EP-A- 289 942**
**EP-A- 0 265 854**
**EP-B- 0 061 113**

(73) Patentinhaber : **BASF Aktiengesellschaft**
**Carl-Bosch-Strasse 38**
**W-6700 Ludwigshafen (DE)**

(72) Erfinder : **Wild, Jochen, Dr.**
**An der Marlach 7**
**W-6705 Deidesheim (DE)**
Erfinder : **Goetz, Norbert, Dr.**
**Schoefferstrasse 25**
**W-6520 Worms 1 (DE)**

EP 0 347 692 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von in 2-, 4- und/oder 6-Position fluorierten Benzaldehyden der allgemeinen Formel I

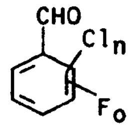

(I)

worin n eine Zahl von 0 bis 4 und o eine ganze Zahl von 1 bis 3 ist, durch Halogenaustausch aus den chlorierten Benzaldehyden der allgemeinen Formel II

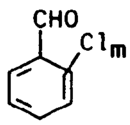

(II)

worin m für eine ganze Zahl von 2 bis 5 steht und worin mindestens ein Chloratom in der 2-, 4- oder 6-Position bezüglich der Aldehydgruppe steht.

Die Herstellung von fluorsubstituierten Benzaldehyden, z.B. 2,6-Difluorbenzaldehyd, ist z.B. in Beilstein, E III 7, 864 oder E IV 7, 561 beschrieben. Danach gelangt man durch Oxidation von 2,6-Difluortoluol mit Brom oder Chlor und anschließender Hydrolyse der Benzalhalogenide zu 2,6-Difluorbenzaldehyd. In J. Med. Chem. 11 (1968) 814-819 und dem britischen Patent 1 080 167 wird zur Synthese von 2,6-Difluorbenzaldehyd die Metallierung von 1,3-Difluorbenzol in der 2-Stellung und die anschließende Formylierung der metallorganischen Verbindung vorgeschlagen. Beide Verfahren gehen aus von fluorierten Vorprodukten, welche sehr aufwendig, in der Regel durch zweifache Schiemann-Reaktion, herzustellen sind.

Folgend den in Z. Obsc. Chim 37 (1967) 171 und DE-OS 31 29 274 beschriebenen Verfahren kann 2,6-Difluorbenzaldehyd gewonnen werden durch Halogenaustausch an 2,6-Dichlorbenzoylchlorid, Überführung des erhaltenen 2,6-Difluorbenzoylfluorids in 2,6-Difluorbenzoylchlorid und anschließende Rosenmundreduktion. Diese Vorgehensweise ist umständlich und außerdem unwirtschaftlich, da bei der Halogenaustauschreaktion zunächst das reaktionsträge Benzoylfluorid entsteht, welches zur weiteren Umsetzung gespalten werden muß. Dadurch geht bei diesem Prozeß ein Fluoratom verloren.

Andere fluorsubstituierte Benzaldehyde, z.B. 2,4-Difluorbenzaldehyd, können auf analogem Weg erhalten werden (Beilstein, E IV.7, 560).

In DE-OS 36 37 156 wird die Halogenaustauschreaktion an 2,4-Dichlorbenzaldehyd mit Kaliumfluorid in Sulfolan beschrieben. Nachteilig an dem Verfahren ist die lange Reaktionsdauer von 15 h.

Der Erfindung lag nun die Aufgabe zugrunde, ein einfaches und wirtschaftliches Verfahren zur Herstellung von fluorsubstituierten Benzaldehyden I durch Halogenaustausch unter Vermeidung der obengenannten Nachteile zur Verfügung zu stellen.

Demgemäß wurde ein Verfahren zur Herstellung von in 2-, 4- oder 6-Position fluorierten Benzaldehyden der allgemeinen Formel I

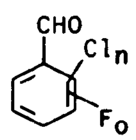

(I)

worin n eine Zahl von 0 bis 4 und o eine ganze Zahl von 1 bis 3 ist, durch Halogenaustausch aus den chlorierten Benzaldehyden der allgemeinen Formel II

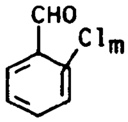

(II)

worin m für eine ganze Zahl von 2 bis 5 steht und worin mindestens ein Chloratom in der 2-, 4- oder 6-Position bezüglich der Aldehydgruppe steht, gefunden, das dadurch gekennzeichnet ist, daß man die chlorsubstituierten Benzaldehyde II mit einem Alkalimetallfluorid in Sulfolan in Gegenwart von Tetramethylammoniumchlorid bei Temperaturen oberhalb 120°C umsetzt und die dabei erzeugten fluorsubstituierten Benzaldehyde I laufend destillativ aus dem Reaktionsgemisch entfernt.

Nach dem beschriebenen Verfahren können bislang nicht zugängliche Benzaldehyde wie 3-Chlor-2,4-difluorbenzaldehyd hergestellt werden.

Der Erfolg des erfindungsgemäßen Verfahrens ist überraschend, da bekanntermaßen Halogenaustauschreaktionen nur dann erfolgreich und mit hoher Raum-Zeit-Ausbeute verlaufen, wenn Chloraromaten, die stark elektronenziehende Gruppen, z.B. Nitro-, Trifluormethyl- oder Nitrilgruppen tragen, eingesetzt werden. An Benzaldehydderivaten sind solche Halogenaustauschreaktionen z.B. in EP-A 61 113 und DE-OS 36 37 156 beschrieben.

Gemäß dem erfindungsgemäßen Verfahren werden Alkalimetallfluoride, insbesondere solche mit stark ionischem Charakter, als Fluorierungsmittel verwendet.

Bevorzugt sind Natrium-, Kalium- und Cäsiumfluorid oder Gemische dieser Fluoride. Besonders bevorzugt ist Kaliumfluorid.

Pro auszutauschendes Halogen verwendet man vorteilhaft mindestens äquimolare Mengen des Alkalimetallfluorids. Die Menge an Alkalimetallfluorid beeinflußt die Produktbildung, wenn mehrere austauschbare Chloratome im Ausgangsmaterial I vorhanden sind. So gelingt es beispielsweise, die Umsetzung des 2,6-Dichlorbenzaldehyds wahlweise in Richtung des 2-Chlor-6-fluorbenzaldehyds oder des 2,6-Difluorbenzaldehyds zu lenken (vgl. Beispiele 1 und 3). Wenn keine weiteren austauschbaren Chloratome im Molekül vorhanden sind oder alle austauschbaren Chloratome durch Fluor ersetzt werden sollen, kann es vorteilhaft sein, einen Überschuß, z.B. 1,1 bis 3 Moläquivalente pro auszutauschendes Äquivalent Chlor zu verwenden. Größere Überschüsse sind möglich, bringen aber keine Vorteile.

Das erfindungsgemäße Verfahren wird besonders vorteilhaft in Gegenwart von Tetramethylammoniumchlorid, welches als Phasentransferkatalysator wirkt, durchgeführt. Die Verwendung anderer Tetramethylammoniumsalze, beispielsweise des Fluorids, Bromids, Jodids, Nitrats, Hydrogensulfats, Perchlorats, Acetats oder Benzoats ist ebenfalls möglich.

Das Tetramethylammoniumchlorid kann in katalytischen Mengen, z.B. 0,1 bis 0,9 mol, stöchiometrischen Mengen oder überschüssigen Mengen, z.B. 1 bis 2 mol, jeweils bezogen auf 1 Mol auszutauschendes Chloratom, verwendet werden. Ein größerer Überschuß ist möglich, bringt jedoch in der Regel keine weiteren Vorteile. Bevorzugt werden 0,2 bis 1 mol Tetramethylammoniumchlorid pro auszutauschendes Chloratom eingesetzt.

Die erfindungsgemäße Umsetzung wird in Gegenwart eines polar aprotischen Lösungsmittels, vorteilhaft eines dipolar aprotischen Lösungsmittels, vorgenommen. Bevorzugt wird Sulfolan (Tetrahydrothiophen-1,1-dioxid) als Lösungsmittel verwendet.

Die Menge an Lösungsmittel wird vorteilhaft so gewählt, daß eine gut rührbare Mischung erhalten wird. Beispielsweise können etwa 100-300 g Lösungsmittel pro 100 g Benzaldehyd II eingesetzt werden. Größere Lösungsmittelmengen sind möglich, aber unwirtschaftlich.

Das Sulfolan soll ebenso wie das Alkalimetallfluorid für das erfindungsgemäße Verfahren weitgehend wasserfrei sein, d.h. sein Wassergehalt sollte weniger als 1 Gew.% betragen. Dieser Grad an Wasserfreiheit des Lösungsmittels kann durch an sich bekannte Methoden erreicht werden, wie Zusatz eines inerten Trockenmittels, Andestillieren der Lösungsmittelmenge, bis durch azeotropes Abdestillieren des Wassers mit einem Teil des Lösungsmittels der geforderte Wassergehalt erreicht wurde oder auch durch azeotrope Destillation des Wassers aus dem Lösungsmittel unter Verwendung eines Schleppmittels für das Wasser. Es ist weiterhin möglich, das Alkalimetallfluorid und das Lösungsmittel vorzulegen, bei denen das Salz oder das Lösungsmittel oder beide einen Wassergehalt oberhalb der genannten Grenze von 1 Gew.% aufweisen, und sodann durch Andestillieren oder durch azeotrope Destillation unter Zusatz eines Schleppmittels den Wassergehalt dieser Salz-Lösungsmittel-Suspension auf das geforderte Maß zu bringen.

Das erfindungsgemäße Verfahren wird bei einer Temperatur oberhalb von 120°C, z.B. 120 bis 300, bevorzugt 150 bis 220, besonders bevorzugt 175 bis 200°C, durchgeführt.

Das erfindungsgemäße Verfahren kann sowohl kontinuierlich als auch diskontinuierlich durchgeführt werden.

Die Reaktionszeiten hängen ab vom Ausgangsstoff II, von den Reaktionstemperaturen, der Katalysatormenge und der Alkalimetallfluoridmenge und betragen bei bevorzugter Durchführung der Umsetzung, z.B. bei der Herstellung von 2,6-Difluorbenzaldehyd, nur 40-90 min (s. Beispiel 1), einer für Halogenaustauschreaktionen erstaunlich kurzen Zeit. Dies ist ein besonderer Vorteil des erfindungsgemäßen Verfahrens. Im Gegensatz zu der in DE-OS 36 37 156 beschriebenen Umsetzung wird eine hohe Raum-Zeit-Ausbeute erreicht (siehe Vergleichsversuche 4 und 5).

Das Zusammengeben der Reaktionspartner und des Lösungsmittels in den Reaktor kann in beliebiger Reihenfolge durchgeführt werden. Vielfach wird das Lösungsmittel vorgelegt und die Reaktionspartner sowie der Katalysator eingetragen. Man kann auch das Alkalimetallfluorid und das Tetramethylammoniumchlorid im Lösungsmittel bei Reaktionstemperatur vorlegen, den Benzaldehyd II, gegebenenfalls gelöst oder in der Schmelze, zudosieren und den entstehenden fluorierten Benzaldehyd I langsam abdestillieren.

Wiederum eine andere Ausführungsform des erfindungsgemäßen Verfahrens besteht darin, daß eine Mischung der obengenannten Komponenten durch einen auf Reaktionstemperatur gebrachten Rohrreaktor gepumpt wird.

Die Isolierung des Wertproduktes und die Rückgewinnung des Lösungsmittels erfolgt bevorzugt auf folgende Weise:

Der leichter flüchtige fluorierte Benzaldehyd wird vom Ausgangsmaterial und dem hochsiedenden Sulfolan durch Destillation, vorzugsweise bei vermindertem Druck, abgetrennt. Dies kann insbesondere bei großen Ansätzen im 10 Mol-Bereich und darüber vorteilhaft bereits während der Umsetzung geschehen.

Es empfiehlt sich, die Destillation im schwachen Vakuum (20-100 mbar) durchzuführen. Das Lösungsmittel kann anschließend z.B. durch Destillation des Sumpfes, gegebenenfalls nach Abfiltrieren der Salze, zurückgewonnen werden.

Die nach dem erfindungsgemäßen Verfahren gewonnenen Benzaldehyde sind wertvolle Ausgangssubstanzen zur Herstellung von Farbstoffen, pharmazeutisch wirksamen Substanzen und Pflanzenschutzmitteln. Insbesondere kann 2,6-Difluorbenzaldehyd zur Synthese von Aroylharnstoffen mit insektizider Wirkung (DE-OS 21 23 236 oder DE-OS 35 42 201) verwendet werden.

Die folgenden Beispiele veranschaulichen das Verfahren.

Herstellung von 2,6-Difluor- und 2-Chlor-6-fluorbenzaldehyd

Beispiel 1

Eine Mischung aus
221 g (1,27 mol) 2,6-Dichlorbenzaldehyd,
150 g (1,37 mol) Tetramethylammoniumchlorid und
292 g (5 mol) Kaliumfluorid in 400 ml Sulfolan
wurde 1 h bei 190°C gerührt. Anschließend wurde die Mischung bei 40 mbar destilliert.

Man erhielt 153,7 g Destillat, bestehend aus:

142 g 2,6-Difluorbenzaldehyd (78,7 % der Theorie),
3,4 g 2-Chlor-6-fluorbenzaldehyd (1,7 % der Theorie),
8,3 g Sulfolan.

Beispiel 2

135 g (0,85 mol) 2-Chlor-6-fluorbenzaldehyd,
116 g (2 mol) Kaliumfluorid,
58 g (0,5 mol) Tetramethylammoniumchlorid und
100 ml Sulfolan wurden bei 190°C gerührt.

Die Destillation (40 mbar) nach 2 h ergab:
87 g 2,6-Difluorbenzaldehyd (72 % der Theorie) und
14 g 2-Chlor-6-fluorbenzaldehyd (10 % der Theorie).

Beispiel 3

Die Mischung aus Beispiel 2 wurde 45 min bei 190°C gerührt. Nach der Destillation erhielt man
75 g 2,6-Difluorbenzaldehyd (62 % der Theorie) und
38 g 2-Chlor-6-fluorbenzaldehyd (28 % der Theorie).

Vergleichsbeispiele ohne Tetramethylammoniumchlorid

Beispiele 4 und 5

Eine Mischung aus

35 g (0,2 mol) 2,6-Dichlorbenzaldehyd und
54 g (0,84 mol) Kaliumfluorid in
100 ml Sulfolan wurde 12 h auf 210°C erhitzt.

Die Aufarbeitung analog Beispiel 1 ergab 20 g einer Mischung bestehend aus:

11 g 2,6-Dichlorbenzaldehyd (31,4 % der Theorie),
7,8 g 2-Chlor-6-fluorbenzaldehyd (24,6 % der Theorie),
1,2 g 2,6-Difluorbenzaldehyd (4,2 % der Theorie).

Eine Mischung aus

35 g (0,2 mol) 2,6-Dichlorbenzaldehyd,
54 g (0,74 mol) Kaliumfluorid und
5,4 g Cäsiumfluorid in
150 ml Sulfolan wurde 2 h auf 190°C erwärmt.

Die Aufarbeitung analog Beispiel 1 ergab 30 g einer Mischung, bestehend aus:

18,1 g 2,6-Dichlorbenzaldehyd (51,8 % der Theorie),
10 g 2-Chlor-6-fluorbenzaldehyd (31,7 % der Theorie),
1,9 g 2,6-Difluorbenzaldehyd (4,2 % der Theorie).

Beispiele 6 und 7

Herstellung von 2,4-Difluorbenzaldehyd

192 g (3,3 mol) Kaliumfluorid,
38,4 g (0,35 mol) Tetramethylammoniumchlorid,
100 g (0,57 mol) 2,4-Dichlorbenzaldehyd und
250 ml Sulfolan wurden 45 min bei 190°C gerührt.

Die Destillation der Mischung (145 bis 160 mbar) ergab:
51 g 2,4-Difluorbenzaldehyd (63 % der Theorie) und
15 g 2,4-Dichlorbenzaldehyd (15 % der Theorie)

Wurde die o.g. Mischung 1 h anstelle von 45 min bei 190°C gerührt, so erhielt man 61 g 2,4-Difluorbenzaldehyd (75 % der Theorie).

Beispiel 8

Herstellung von 3-Chlor-4-fluorbenzaldehyd

146 g (2,5 mol) Kaliumfluorid,
75 g (0,68 mol) Tetramethylammoniumchlorid und
200 ml Sulfolan wurden bei 190°C mit
90 g (0,51 g) 3,4-Dichlorbenzaldehyd versetzt.

Die Mischung wurde 30 min bei 190°C gerührt und anschließend im Vakuum (50 mbar) destilliert. Man erhielt 56 g 3-Chlor-4-fluorbenzaldehyd (69 % der Theorie).

Beispiel 9

Herstellung von 3-Chlor-2,4-difluorbenzaldehyd

290 g (5 mol) Kaliumfluorid,
85 g (0,77 mol) Tetramethylammoniumchlorid und
223,5 g (1,06 mol) 2,3,4-Trichlorbenzaldehyd in
425 ml Sulfolan wurden 1 h bei 190°C gerührt.

Die Destillation ergab 118 g 3-Chlor-2,4-difluorbenzaldehyd (63 % der Theorie).

Beispiel 10

Herstellung von 3-Chlor-2-fluorbenzaldehyd

146 g (2,5 mol) Kaliumfluorid und
75 g (0,68 mol) Tetramethylammoniumchlorid in
200 ml Sulfolan wurden bei 190°C mit
90 g (0,51 mol) 2,3-Dichlorbenzaldehyd versetzt.

Die Mischung wurde 2 h bei 170°C und 1 h bei 190°C gerührt.
Die Destillation ergab 549 g (67 % der Theorie) 3-Chlor-2-fluorbenzaldehyd.

**Patentansprüche**

1. Verfahren zur Herstellung von in 2-, 4- oder 6-Position fluorierten Benzaldehyden der allgemeinen Formel I

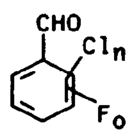

(I)

worin n eine Zahl von 0 bis 4 und o eine ganze Zahl von 1 bis 3 ist, durch Halogenaustausch aus den chlorierten Benzaldehyden der allgemeinen Formel II

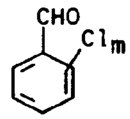

(II)

worin m für eine ganze Zahl von 2 bis 5 steht und worin mindestens ein Chloratom in der 2-, 4- oder 6-Position bezüglich der Aldehydgruppe steht, dadurch gekennzeichnet, daß man die chlorsubstituierten Benzaldehyde II mit einem Alkalimetallfluorid in Sulfolan in Gegenwart von Tetramethylammoniumchlorid bei Temperaturen oberhalb 120°C umsetzt und die dabei erzeugten fluorsubstituierten Benzaldehyde I laufend destillativ aus dem Reaktionsgemisch entfernt.

2. Verfahren nach Anspruch 1, dadurch gekennzeichnet, daß man die Umsetzung bei Temperaturen von 150 bis 300°C vornimmt.

3. 3-Chlor-2,4-difluorbenzaldehyd.

## Claims

1. A process for preparing a 2-, 4- or 6-fluorinated benzaldehyde of the general formula I

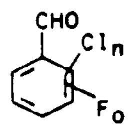

(I)

where n is from 0 to 4 and o is an integer from 1 to 3, by halogen replacement from a chlorinated benzaldehyde of the general formula II

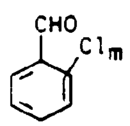

(II)

where m is an integer from 2 to 5 and at least one chlorine atom is in the 2-, 4- or 6-position relative to the aldehyde group, which comprises reacting the chlorine-substituted benzaldehyde II with an alkali metal fluoride in sulfolane in the presence of tetramethyl-ammonium chloride at above 120°C and continuously removing the resulting fluorine-substituted benzaldehyde I from the reaction mixture by distillation.

2. A process as claimed in claim 1, wherein the reaction is carried out at from 150 to 300°C.

3. 3-Chloro-2,4-difluorobenzaldehyde.

## Revendications

1.- Procédé de préparation de benzaldéhydes fluorés en position 2, 4 ou 6 de formule générale

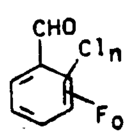

(I)

dans laquelle n est un nombre de 0 à 4 et o est un nombre entier de 1 à 3, par échange d'halogènes à partir

des benzaldéhydes chlorés de formule générale II

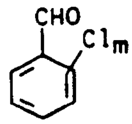

(II)

dans laquelle m est mis pour un nombre entier de 2 à 5 et dans laquelle un atome de chlore au moins est en position 2, 4 ou 6 par rapport au groupement aldéhyde, caractérisé en ce qu'on fait réagir les benzaldéhydes chlorosubstitués II avec un fluorure de métal alcalin dans du Sulfolane en présence de chlorure de tétraméthylammonium à des températures supérieures à 120°C, et on extrait du mélange réactionnel, par distillation continue, les benzaldéhydes fluorosubstitués I ainsi produits.

2.- Procédé selon la revendication 1, caractérisé en ce qu'on conduit la réaction à des températures de 150 à 300°C.

3.- 3-chloro-2,4-difluorobenzaldéhyde